(19) Europäisches Patentamt / European Patent Office / Office européen des brevets

(11) **EP 3 929 931 A1**

(12) **EUROPEAN PATENT APPLICATION**

(43) Date of publication:
**29.12.2021 Bulletin 2021/52**

(51) Int Cl.:
***G16H 20/60*** *(2018.01)*

(21) Application number: **20182440.6**

(22) Date of filing: **26.06.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **JIN, Yafang**
  **5656 AE Eindhoven (NL)**

• **KUI, Xiao Yun**
  **5656 AE Eindhoven (NL)**
• **XIAO, Weimin**
  **5656 AE Eindhoven (NL)**
• **MA, Hehe**
  **5656 AE Eindhoven (NL)**
• **SUN, WEN**
  **5656 AE Eindhoven (NL)**

(74) Representative: **Kapoor, Pavan Puneet**
**Philips Domestic Appliances**
**IP Department**
**High Tech Campus 42**
**5656 AE Eindhoven (NL)**

(54) **DISH NUTRITIONAL VALUE ESTIMATION**

(57)    A method (100) is disclosed of estimating a nutritional content of a dish comprising a plurality of ingredients. The method comprises retrieving (107, 109) a list of ingredients of the dish, e.g. from a recipe database; retrieving nutritional information for each of said listed ingredients from an ingredient database; receiving (111) a digital image of said dish; evaluating (113) the digital image based on the retrieved list of ingredients using ingredient identifiers for each of the ingredients in said list to identify regions within the digital image occupied by said ingredients; estimating the relative amounts of the ingredients in said dish recognized in said digital image from said identified regions; estimating (115) the nutritional content of said dish using said estimated relative amounts and the retrieved nutritional information; and generating (117) an output comprising the estimated nutritional content of said dish. Also disclosed is a computer program product for implementing such a method and an electronic device configured by such a computer program product.

FIG. 4

**Description**

FIELD OF THE INVENTION

The present invention relates to a method of estimating a nutritional content of a dish comprising a plurality of ingredients, a computer program product for implementing such a method and an electronic device comprising the computer program product.

BACKGROUND OF THE INVENTION

In personalized nutrition, the goal is to provide a person with balanced nutrition tailored to the needs of the person, e.g. to control the (daily) intake of food in terms of nutritional values, such as energy intake, fat intake, carbohydrate intake, protein intake and so on. In order to provide such a person with a tailored diet, it is important to gain insights into the eating habits of the person. This therefore requires the person to keep a log or diary of the eating habits over a prolonged period of time, such that the information collected in this manner facilitates the assessment of the person's eating habits and the compilation of a dietary program based thereon.

Unfortunately, most people experience the keeping of such dietary records as tedious and cumbersome such that compliance with such records keeping requirements is typically low, thereby hampering the generation of an appropriate diet plan for such persons. In order to make such a record-keeping task less burdensome, software apps are available that allow its users to extract the required data pertaining to a consumed dish from a recipe database or the like. To this end, the user may provide a name or an image of the dish, which information is used as an identifier to identify the dish in the recipe database and obtain the recipe information from the database, e.g. for the purpose of adding this information to an electronic dietary diary of the user.

For example, US 2016/110423 A1 discloses a cooking recipe information provision device including a recipe name obtaining unit for obtaining a recipe name inputted by a user; a recipe extraction unit for extracting a plurality of recipes, based on the recipe name obtained from a recipe information database for storing a plurality of recipes, ingredients used in the respective recipes, and amounts of the respective ingredients so as to be correlated to each other; and a display data generation unit for extracting a recipe, based on the amount of the main ingredient, from among the plurality of recipes extracted, and generating display data indicating information on the extracted recipe, in order to obtain the correct recipe from such a database based on a comparison between the actual amount of the main ingredient and the amount of this ingredient in such recipes.

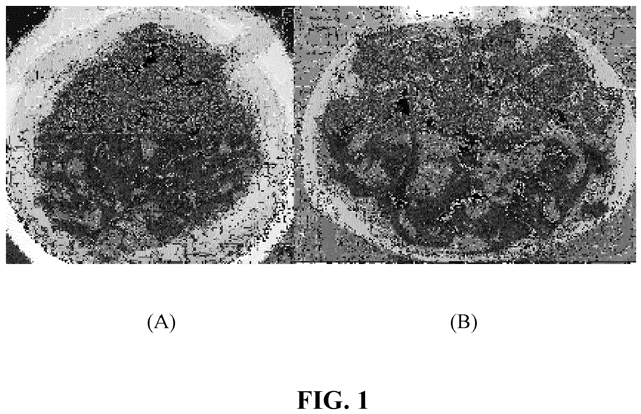

However, this approach is based on the assumption that the person preparing the dish has not altered its recipe, which in reality rarely is the case because people tend to make changes to recipes in order to meet their own personal tastes. This is for instance demonstrated in FIG. 1, which shows two images of a stir-fried and meat dish prepared by two different people from the same recipe. It can be clearly seen that dish (A) in the left-hand image contains more meat, whereas dish (B) in the right-hand image contains more peppers. Therefore, use of the dietary information associated with the recipe from such a recipe database can lead to an inaccurate estimation of the nutritional value of the particular dish due to changes made to this recipe. Hence, there exists a need for a more accurate nutritional value estimation for such dishes.

SUMMARY OF THE INVENTION

The present invention seeks to provide a method of estimating the nutritional content of a dish comprising a plurality of ingredients in which the ingredient composition of the dish can be more accurately estimated.

The present invention further seeks to provide a computer program product for implementing such a method on an electronic device such as a computer or the like.

The present invention yet further seeks to provide an electronic device equipped with such a computer program product.

According to an aspect, there is provided a method of estimating a nutritional content of a dish comprising a plurality of ingredients, the method comprising retrieving a list of ingredients of the dish; retrieving nutritional information for each of said listed ingredients from an ingredient database; receiving a digital image of said dish; evaluating the digital image based on the retrieved list of ingredients using ingredient identifiers for each of the ingredients in said list to identify regions within the digital image occupied by said ingredients; estimating the relative amounts of the ingredients in said dish recognized in said digital image from said identified regions; estimating the nutritional content of said dish using said estimated relative amounts and the retrieved nutritional information; and generating an output comprising the estimated nutritional content of said dish.

The present invention is based on the insight that image recognition techniques may be deployed in order to partition a digital image of a dish into the various ingredients within the dish such that the actual composition of the dish can be more accurately estimated by the evaluation of the partitioned digital image to obtain an estimation of the actual relative amounts of each ingredient within the dish such that based on the nutritional information of each ingredient as obtained from a nutritional database the nutritional value of the dish as a whole an accurate estimation of the nutritional value of the dish can be derived from the relative amounts of its main ingredients.

Preferably, identifying regions within the digital image occupied by said ingredients and estimating the relative amounts of the ingredients in said dish recognized in said digital image from said identified regions comprises segmenting the digital image into segments occupied by a single ingredient; grouping the segments corresponding to the same ingredient into an ingredient segment group; and determining the total area of the dish covered by each ingredient segment group; and wherein determining the relative amounts of said individual ingredients is based on the determined total areas of the ingredient segment groups. In this manner, the total area occupied by a single ingredient the dish can be directly correlated to its relative amount within the dish, as this relative amount typically relates to the total area occupied by its ingredient segment group.

In a further refinement, the method further comprises obtaining density information for each ingredient; converting the determined total areas of each of the ingredient segment groups into total volumes; and wherein determining the relative amounts of each individual ingredient is based on the product of the total volume of the its ingredient segment group and its density. This further improves the accuracy of the estimation of the relative amounts for each ingredient, in particular in scenarios in which different ingredients have (significantly) differing densities.

Such density information for example may be obtained from a recipe database. The density information may correspond to a particular cooking method of the dish to further improve the accuracy of the estimated relative amounts of the ingredients within the dish as different cooking methods of a particular ingredient can lead to different densities of that ingredient in the prepared dish.

The list of ingredients as used in the method may be obtained in a number of ways. In a preferred embodiment, retrieving the list of ingredients comprises receiving a dish identifier for said dish; and retrieving a recipe of said dish from a recipe database upon matching said recipe to said dish identifier, said recipe comprising said list of ingredients and default amounts of said ingredients within the dish, and updating said default amounts based on the estimated relative amounts for said ingredients. This has the advantage that the recipe information insofar related to the list of (main) ingredients can be updated using the estimated amounts of these ingredients as obtained with the image recognition algorithm.

The aforementioned dish identifier may be a dish name, e.g. provided in text form or in spoken form as recognized by a speech recognition algorithm, in which case the dish name may be matched to a recipe in the recipe database by comparing the user-provided dish name to the dish names stored in the recipe database, or may be a dish image provided by the user in which case the dish image may be matched to a recipe in the recipe database by comparing the user-provided dish image to the dish images stored in the recipe database.

Where the recipe of said dish comprises default relative amounts of one or more seasonings for said dish, the method may further comprise estimating the nutritional content of said dish based on the estimated relative amounts of the ingredients and the default relative amounts of one or more seasonings for said dish. This has the further advantage of being able to factor in additional information present in the recipe and not obtainable from the image evaluation, such as seasoning, cooking method-related additives such as amounts of oil for frying or the like, as this additional information can be obtained from the recipe information to obtain particularly accurate nutritional value information for the actual dish.

This may be further refined by the method further comprising receiving a user input updating the relative amount of at least one of said one or more seasonings for said dish; and estimating the nutritional content of said dish based on the updated relative amounts of the ingredients, the user-updated amounts of seasonings for said dish and the default relative amounts of the remaining seasonings for said dish to further improve the accuracy of the nutritional value estimate of the dish.

In case an ingredient is not recognised by the image recognition algorithm, the method may further comprise estimating the relative amount of a single unidentified ingredient in the evaluation of said digital image from a difference between the total area occupied by the dish in said digital image and the total area covered by the identified ingredient segment groups within said dish such that an accurate estimate of the nutritional value of the dish can still be obtained.

Where the image recognition algorithm fails to recognise multiple ingredients, the method may further comprise estimating the relative amount of multiple unidentified ingredients in the evaluation of said digital image from a difference between the total area occupied by the dish in said digital image and the total area covered by the identified ingredient segment groups within said dish; and a ratio between the amounts of the multiple unidentified ingredients in the recipe such that an accurate estimate of the nutritional value of the dish can still be obtained.

In an alternative embodiment, retrieving the list of ingredients comprises parsing a received dish name for individual ingredients in said dish name and generate the list of ingredients from said parsing. This for instance may be advantageous in scenarios in which the provided dish name cannot be matched to a database entry in the recipe database.

The image recognition algorithm may use any suitable ingredient identifiers to identity the listed ingredients in the dish image. For example, the ingredient identifiers may include at least one of colour, texture and shape of said ingredient.

The estimated nutritional content of the dish may take any suitable form, and may be at least one of total energy content, fat content, carbohydrate content and protein content. The nutritional content may be expressed in any suitable manner, such as in a relative manner, i.e. a content amount per unit weight of the dish, e.g. per 10g, 50g or 100g of the dish, or any other suitable unit weight.

According to another aspect, there is provided a computer program product comprising a computer readable storage medium having computer readable program instructions embodied therewith for, when executed on a processor arrangement of an electronic device, cause the processor arrangement to implement the method of any of the herein described embodiments. Such a computer program product may therefore be used to configure or program an electronic device such that the electronic device can implement this method.

According to yet another aspect, there is provided an electronic device comprising a processor arrangement and the aforementioned computer program product, wherein the processor arrangement is adapted to execute said computer readable program instructions, thereby providing an electronic device such as a computer, smart phone, tablet or the like adapted to implement the nutritional content estimating method according to embodiments of the present invention.

Such an electronic device may further comprise at least one of a camera communicatively coupled to the processor arrangement and arranged to capture said digital image; and a display device communicatively coupled to the processor arrangement and arranged to display said output. For example, the electronic device may be a portable device such as a smart phone, a tablet computer or the like.

BRIEF DESCRIPTION OF THE DRAWINGS

Embodiments of the invention are described in more detail and by way of non-limiting examples with reference to the accompanying drawings, wherein:

FIG. 1 depicts images of different interpretations of dishes prepared from a single recipe;
FIG. 2 schematically depicts an electronic device according to an example embodiment;
FIG. 3 schematically depicts a system including an electronic device according to an embodiment;
FIG. 4 is a flowchart of a method according to an embodiment;
FIG. 5 depicts images of three dishes with the same ingredients in different relative amounts that were evaluated in accordance with a method according to an embodiment; and
FIG. 6 depicts images of two more dishes with the same ingredients in different relative amounts that were evaluated in accordance with a method according to an embodiment.

DETAILED DESCRIPTION OF THE EMBODIMENTS

It should be understood that the Figures are merely schematic and are not drawn to scale. It should also be understood that the same reference numerals are used throughout the Figures to indicate the same or similar parts.

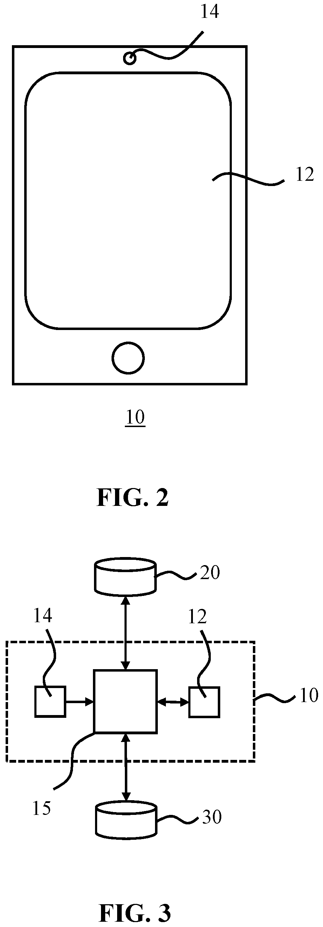

FIG. 2 schematically depicts an example embodiment of an electronic device 10 for implementing embodiments of the method 100 of the present invention. The electronic device 10 may comprise a display screen 12, which for example may be a touchscreen also acting as a user interface of the electronic device 10 and one or more cameras 14 such as a front facing camera and/or a rear facing camera, which camera may be used to capture a digital image of a dish of which the contents are to be evaluated in order to obtain an estimate of the nutritional content of the dish. For example, the electronic device 10 may be a smart phone or a tablet computer or a comparable portable electronic device although it should be understood that the electronic device 10 may take any suitable shape, such as a personal computer, laptop computer, server or the like in which the digital image of the dish to be evaluated may be received from a separate electronic device over a data communication link between the electronic device 10 and the separate electronic device.

As shown in FIG. 3, the electronic device 10 typically comprises a processor arrangement 15, which processor arrangement may be communicatively coupled to the display screen 12 and the one or more cameras 14 if present. For instance, the processor arrangement 15 may be arranged to control the operation of the display screen 12 and the one or more cameras 14. In the context of the present application, where reference is made to a processor arrangement 15, it should be understood that this is intended to cover embodiments in which the electronic device 10 comprises a single processor 15 as well as multiple processors or processor elements, e.g. cores, implementing such a processor arrangement. The processor arrangement 15 may be communicatively coupled to a recipe database 20 and an ingredient database 30 over any suitable data communication link, e.g. a 3G, 4G or 5G mobile communications link, a wired or wireless Internet connection and so on. To this end, the electronic device 10 may comprise one or more network interfaces (not shown) to facilitate such data communication between the processor arrangement 15 and either one of the recipe database 20 and the ingredient database 30. As such network interfaces are entirely routine, they are not explained in further detail for the sake of brevity only. It should be understood that any suitable implementation of such network interfaces may be deployed within the electronic device 10. Also, although FIG. 3 depicts the recipe database 20 and ingredient database 30 as separate database entities, it should be understood that this is by way of non-limiting example only and that embodiments in which the information in the recipe database 20 and the ingredient database 30 is contained within a single database structure are equally feasible.

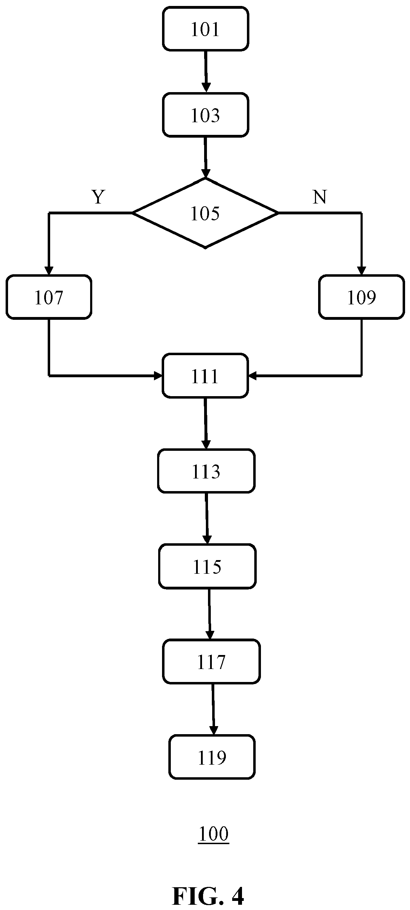

The electronic device 10 is equipped with a computer program product implementing one or more embodiments of the method 100 of the present invention, a flowchart of which is depicted in FIG. 4. The method starts in operation 101, after which the method progresses to operation 103 in which the processor arrangement 15 of the electronic device 10 receives a dish identifier from a user of the electronic device 10. The dish identifier is for the purpose of finding a matching recipe for the dish in the recipe database 20, which recipe typically contains a list of ingredients and their amounts in the recipe, and optionally also at least one of defaults amounts of seasoning in the dish, condiments such as oil used in the dish and cooking method of the dish. The amounts may be relative amounts, e.g. may be expressed as a percentage or as an amount per unit weight of the dish, e.g. X grams of an ingredient per 100g of the dish, in which X is a positive real number not exceeding 100. In such a scenario, all listed ingredients, seasonings and condiments typically add up to the unit amount of the dish, e.g. where the unit amount is 100g, the specified amounts of these various ingredients, seasonings and condiments add up to 100g.

The dish identifier may take any suitable shape. In an embodiment, the dish identifier may be a dish name, e.g. stir-fry pork and peppers, which may be provided to the processor in textual format, e.g. through a user interface such as a touchscreen display device 12, keypad and so on, or in spoken form, e.g. through a microphone (not shown) communicatively coupled to the processor arrangement 15, in which case the processor arrangement 15 may deploy speech recognition algorithms to recognize the dish name from the received speech. Alternatively, the dish identifier may be provided as a digital image of the dish. Next, the processor arrangement 15 contacts the recipe database 20 in operation 105 and provides the recipe database 20 with the dish identifier in order to obtain a recipe matching the dish identifier from the recipe database 20 in operation 107 if the recipe database was able to match the dish identifier to a particular recipe. Alternatively, such matching of the dish identifier to a recipe in the recipe database 20 may be performed on the processor arrangement 15 instead. In case of the dish identifier producing multiple matches, the processor arrangement 15 may control an output device such as the display screen 12 and provide the user of the electronic device 10 with a list of matching recipes, such that the user can select the appropriate recipe from this list. In either case, a list of ingredients in the dish to be evaluated can be retrieved from the matched recipe.

In case no match was found for the provided dish identifier in the recipe database 20, the method 100 proceeds to operation 109 in which the processor arrangement parses the dish identifier (which then must be a text-based dish identifier) in order to extract the list of ingredients from the parsing results. For example, where the dish identifier is 'stir-fried pork and peppers', the processor arrangement obtains 'pork' and 'peppers' from the parsing results. Such parsing may extract N ingredients from the textual dish identifier, in which case the processor arrangement may assign an initial amount of M/N grams to each identified ingredient, in which N and M are positive integers and M is the unit weight of the dish. Optionally, the parsing result of the dish identifier may further identify the cooking method of the dish, such as stir-frying in the above example, which may be used to obtain nutritional information for each ingredient from the ingredient database 30 as a function of this cooking method, as will be explained in further detail below.

For the thus obtained list of ingredients, the processor arrangement 15 may obtain the nutritional information for each ingredient on the list from the ingredient database 30 and compile an information table such as Table 1 shown below, which contains the information obtained from the recipe database 20 (or parsing operation), e.g. the list of ingredients, their default amounts and cooking method if available, and the nutritional values of each of the ingredients, energy content, fat content, protein content, carbohydrate content, vitamin content, mineral content and so on, e.g. as a function of the cooking method, as retrieved from the ingredient database 30. Preferably, the information obtained from the ingredient database 30 also contains density information for at least the main ingredients of the dish such that the actual weight fraction of such ingredients within the dish can be calculated more accurately. This is advantageous, because the density of an ingredient can dramatically change when being prepared, e.g. cooked, boiled, fried, and so on. For example, the density of raw spinach is 0.08 g/ml, whereas the density of cooked spinach is 1.046 g/ml. Of course, in the absence of such density information, a default density for each ingredient may be assumed, or the ingredient density may not be taken into consideration altogether.

Table 1

| | Name | Default weight proportion (%) | Nutrients | | | Density (g/mL) |
|---|---|---|---|---|---|---|
| | | | Energy (Kcal/100g) | Fat (g/100g) | ... | |
| Cooking method | (e.g. stir-frying, boiling) | | | | | |

(continued)

| | Name | Default weight proportion (%) | Nutrients | | | Density (g/mL) |
| | | | Energy (Kcal/100g) | Fat (g/100g) | ... | |
|---|---|---|---|---|---|---|
| **Ingredients** | a | $W_a$ | $E_a$ | $F_a$ | ... | $D_a$ |
| | b | $W_b$ | $E_b$ | $F_b$ | ... | $D_b$ |
| | c | $W_c$ | $E_c$ | $F_c$ | ... | $D_c$ |
| | ... | ... | ... | ... | ... | ... |
| | n | $W_n$ | $E_n$ | $F_n$ | ... | $D_n$ |
| **Seasonings (e.g., cooking oil, sugar, salt)** | S1 | $W_{s1}$ | $E_{s1}$ | $F_{s1}$ | ... | |
| | S2 | $W_{s2}$ | $E_{s2}$ | $F_{s2}$ | ... | |
| | ... | ... | ... | ... | ... | |
| | Sn | $W_{sn}$ | $E_{sn}$ | $F_{sn}$ | ... | |
| **Total** | | 100% | | | ... | |

The weight fractions W of the default weight of the main ingredients and seasoning preferably add up to 100%:

$$\sum_{i=1}^{n} Wi + \sum_{j=1}^{sn} Wj = 100\%,$$ in which n is the number of ingredients, and sn is the number of seasonings. Regarding the seasonings, it is noted that sugar and cooking oil typically are the most important seasonings to be taken into account due to their relatively high impact on the nutritional value of a dish, but other seasonings of course may be considered as well. The information in Table 1 may be used to calculate the default nutritional content of the dish based on the default weight fractions obtained as explained above. For example, the default total energy of the dish may be calculated

$$\text{Default Total Energy} = \sum_{i=1}^{n} Wi * Ei + \sum_{j=1}^{sn} Wj * Ej.$$ as follows: Obviously, similar expressions may be used to calculate other nutritional values. For example, for the default total fat content, the above equation is amended by replacing $E_i$ with $F_i$ and $E_j$ with $F_j$. This is entirely straightforward and will be explained in further detail for the sake of brevity only.

In line with the teachings of the present invention, the method 100 aims to update the default weight fractions of the main ingredients of the dish based on an evaluation of a digital image of the dish. To this end, the processor arrangement 15 receives a digital image of the dish to be evaluated in operation 111, for example from the camera 14 or from a camera in a remote electronic device that is communicatively coupled to the electronic device 10. In operation 113, the processor arrangement 15 evaluates the received digital image of the dish based on the list of ingredients of the dish as received in operations 107 or 109. To this end, the processor arrangement 15 may deploy image recognition algorithms of an ingredient recognition model, which algorithms rely on ingredient identifiers for each ingredient in the received list of ingredients to recognize each ingredient in the digital image. Such ingredient identifiers may include but are not limited to ingredient shape, ingredient size, ingredient texture, ingredient colour and so on. More specifically, the ingredient recognition model may perform a similarity evaluation for each region in the dish occupied by a single ingredient using a similarity algorithm that may be a supervised learning model such as a multiple class support vector machine, or using a neural network or the like to develop an ingredient classification model. Such an ingredient recognition model may developed in any suitable manner, e.g. using a combination of prior knowledge such as training data and feature information extracted from such digital dish images, e.g. by calculating similarities between the training data and the identified ingredients to find a best match to the ingredients in the training data for each ingredient recognized in the digital image.

In an embodiment, the processor arrangement 15 segments the received digital image of the dish into regions occupied by a single ingredient, e.g. based on boundary recognition techniques, which are well known per se and are therefore not explained in further detail for the sake of brevity only. The thus identified regions may each be evaluated using the aforementioned similarity evaluation of the ingredient recognition model, such that each region is assumed to be a particular ingredient based on a best match to one of the ingredient identifiers in the ingredient recognition model. The thus identified regions or segments of the digital image may be grouped into ingredient segment groups containing all regions of segments belonging to a particular ingredient. In this manner, the total area within the dish occupied by a single ingredient can be derived from the total dish area occupied by its corresponding ingredient segment group, e.g.

6

by counting the total number of pixels in the dish area of the digital image occupied by such an ingredient segment group such that the relative amount or weight W of a particular ingredient i may be estimated as Wi = Pi/Pt, in which Pi is the total number of pixels within its ingredient segment group and Pt is the total number of pixels in the dish area within the digital image.

In this manner, the default weight fractions of the main ingredients of the dish may be updated based on the aforementioned digital image analysis such that the nutritional content of the dish may be calculated by the processor arrangement 15 in operation 115 based on the updated weight fractions using the earlier described equations. Optionally, the default weight fractions of at least some of the seasoning may also be manually updated by a user, e.g. through a user interface such as the display 12 communicatively coupled to the processor arrangement 15, in which case the actual nutritional content of the dish may be calculated based on the updated weight fractions of the main ingredients as well as the updated weight fractions of the seasoning. If not all seasoning amounts have been updated, then the processor arrangement 15 may calculate the nutritional content of the dish based on the updated relative amounts (weight fractions) of the ingredients, the user-updated amounts (weight fractions) of seasonings for the dish and the default relative amounts (weight fractions) of the remaining seasonings for the dish.

Where density information is provided for the (main) ingredients, the calculation of the nutritional content in operation 115 by the processor arrangement 15 may be further refined as follows. For example, the ingredient recognition model may assume that the area ratio of the various ingredients within the dish equals the volume ratio of these ingredients, such that the weight proportion or fraction of these ingredients may be estimated using the available density information for these ingredients, as summarized in Table 2:

Table 2

| | Name | Default weight proportion (%) | Volume ratio, % (=area ratio by image recognition) | Density (g/mL) | Calibrated weight proportion (%) |
|---|---|---|---|---|---|
| | a | $W_a$ | $V_a$ | $D_a$ | $W'_a$ |
| | b | $W_b$ | $V_b$ | $D_b$ | $W'_b$ |
| Ingredients | c | $W_c$ | $V_c$ | $D_c$ | $W'_c$ |
| | ... | ... | ... | ... | ... |
| | n | $W_n$ | $V_n$ | $D_n$ | $W'_n$ |
| | S1 | $W_{s1}$ | | | $W_{s1}$ |
| Seasonings (e.g., cooking oil, sugar, salt) | S2 | $W_{s2}$ | | | $W_{s2}$ |
| | ... | ... | | | ... |
| | Sn | $W_{sn}$ | | | $W_{sn}$ |
| Total | | 100% | | | 100% |

For the n ingredients, it is assumed that $\sum_{i=1}^{n} Vi = 100\%$, that is, these ingredients form the total volume

$$W'_n = \frac{\sum_{i=1}^{n} Wi}{100\%} * \frac{Vn*Dn}{\sum_{i=1}^{n} Vi*Di}.$$

of the dish. Then, the calibrated weight proportion of the ingredients may be calculated by
As will be readily understood, the calibrated weight proportion of the main ingredients is their actual weight proportion within the dish when taking their density in the prepared dish into consideration. The density of the seasonings may be ignored, due to the relatively small amounts of such seasonings in the dish and the fact that the density of such seasonings does not significantly change during preparation of the dish. Consequently, when using such calibrated weight proportions

$$\sum_{i=1}^{n} W'i + \sum_{j=1}^{sn} Wj = 100\%;$$

for the main ingredients, the following equation preferably should hold: in other words, the combined calibrated weight proportions for the main ingredients and weight proportions of the seasonings should add up to 100% to provide an accurate estimate of the nutritional content of the dish in operation 115 based on the calibrated weight proportions for the main ingredients and weight proportions of the seasonings, e.g., the calibrated total

$$\sum_{i=1}^{n} W'i * Ei + \sum_{j=1}^{sn} Wj * Ej.$$

energy of the dish may be calculated as follows: Calibrated Total Energy =

Upon calculation of the nutritional content of the dish in this manner in operation 115, the method 100 proceeds to operation 117 in which the processor arrangement 15 generates an output containing the calculated nutritional content of the dish. Such an output for example may be stored in a digital dietary diary or log of the user of the electronic device 10 and/or may be used to control the display 12 to display the calculated nutritional content of the dish. The output may take any suitable shape or form, and may be used for any suitable purpose. Upon generation of the output in operation 117, the method 100 terminates in operation 119.

Now, upon returning to operation 113, the evaluation of the digital image may in exceptional cases lead to not every ingredient being recognized within the digital image, i.e. the similarity algorithm cannot match a particular instance of an ingredient within the dish to one of its ingredient identifiers corresponding to the received list of ingredients. This for instance may occur where a different variety of an ingredient has been used or where the ingredient has been prepared in an unusual manner, leading to an unexpected appearance of the ingredient. A distinction can be made between two scenarios.

In a first scenario, the similarity algorithm fails to recognize any instances of one or more ingredients. If this is the case for a single ingredient, the actual weight proportion of the missing ingredient may simply be assumed to equal the total area occupied by unrecognized ingredients within the dish, that is, the processor arrangement 15 may estimate the relative amount of a single unidentified ingredient in the evaluation of the digital image from the difference between the total area occupied by the dish in the digital image and the total area covered by the identified ingredient segment groups within the dish. If multiple ingredients cannot be recognized by the similarity algorithm, the processor arrangement 15 may estimate the relative amount of the multiple unidentified ingredients in the evaluation of the digital image from the difference between the total area occupied by the dish in the digital image and the total area covered by the identified ingredient segment groups within said dish and the ratio between the amounts of the multiple unidentified ingredients in the recipe. For example, for two unrecognized ingredients A and B being present in the recipe in a weight ratio of 2:1, the processor arrangement 15 may assume that the unaccounted for area within the dish is occupied by these unrecognized ingredients in this weight ratio, e.g. when 30% of the dish area is unaccounted for, the processor arrangement will assign 20% of the dish area to ingredient A and 10% of the dish area to ingredient B.

In a second scenario, some but not all instances of a particular ingredient are recognized by the similarity algorithm. Again, this will lead to part of the dish area being unaccounted for in terms of ingredient identification by the similarity algorithm. In a first example embodiment, it may be assumed by the processor arrangement 15 that for the N main ingredients in the dish there is an equal likelihood of an instance of such an ingredient not being recognized by the similarity algorithm such that the determined ingredients ratio is in fact correct and does not need amending. In an alternative example embodiment, the ingredient recognition model may include knowledge about the likelihood of an instance of particular ingredient not being recognized by the similarity algorithm. For example, for ingredient A this likelihood may be 10% and for ingredient B this likelihood may be 20%, i.e. it is twice as likely that ingredient B is not recognized compared to ingredient A. In this case, the area of the dish covered by unrecognized ingredients may be assigned to the ingredients based on this knowledge, e.g. where 15% of the area of the dish is occupied by unrecognized ingredients, 10% of the dish area may be assigned to ingredient B and 5% may be assigned to ingredient A based on the knowledge about the likelihood of such ingredients not being recognized by the similarity algorithm.

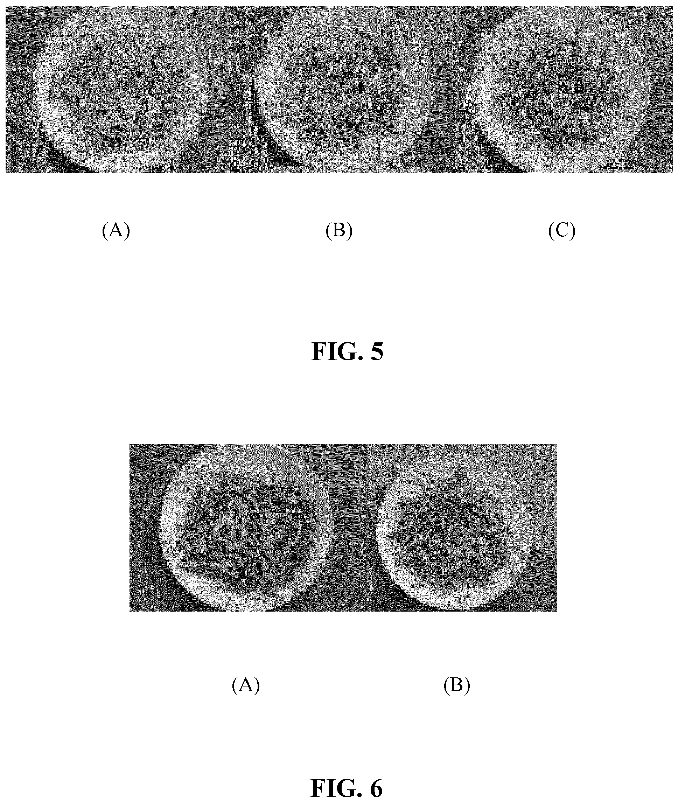

Next, proof of concept for the method 100 will be provided by the following example experiments. It should be understood that these examples are not intended to limit the scope of the present invention in any way, shape or form. In a first experiment, three dishes as shown in FIG. 5 were prepared with stir-fried pepper and meat with different proportions of pepper and meat. The experimental details are listed in Table 3.

Table 3

| | Pepper (g) | Pork (g) | Cooking oil (g) | Salt (g) | Total Energy (Kcal/100g) |
|---|---|---|---|---|---|
| Stir-fried pepper and meat (dish A) | 100 | 50 | 5 | 1.5 | 88 |
| Stir-fried pepper and meat (dish B) | 75 | 75 | 5 | 1.5 | 108 |
| Stir-fried pepper and meat (dish C) | 50 | 100 | 5 | 1.5 | 127 |

Table 4 depicts the recipe and nutritional information as retrieved by the processor arrangement 15 from the recipe database 20 and the ingredient database 30. Based on the default weight proportions from the recipe database 20, the default total energy for the dish is 108 Kcal/100g. As will be immediately apparent, this default total energy is inaccurate for dishes A and C due to the altered ratios of meat and pepper in these dishes.

Table 4

| | Name | Default weight (g) | Default weight proportion (%) | Nutrients | | Density (g/mL) |
|---|---|---|---|---|---|---|
| | | | | Energy (Kcal/100g) | ... | |
| Ingredients | Pepper | 150 | 48% | 22 | ... | 0.51 |
| | Pork | 150 | 48% | 143 | ... | 0.7 |
| Seasonings (e.g., cooking oil, sugar, salt) | Cooking oil | 10 | 3% | 899 | ... | |
| | Salt | 3 | 1% | 0 | ... | |
| Total | | 313 | 100% | 108 | ... | |

In this experiment, digital images of the dishes A-C were obtained with a smart phone and the area ratio of different ingredients in these images was analyzed using the similarity algorithm by determining the number of pixels occupied by each ingredient in the dish area of these images. The results are listed in Table 5.

Table 5

| | Dish A | Dish B | Dish C |
|---|---|---|---|
| Area ratio of pepper (%) | 69% | 56% | 47% |
| Area ratio of meat (%) | 31% | 44% | 53% |
| Total | 100% | 100% | 100% |

As previously explained, by assuming that the area ratio of different ingredients is equal to the volume ratio, the weight proportion of different ingredients by weight can be estimated using the density information for the corresponding ingredients as obtained from the ingredient database 30. The calculation results for the energy content of these dishes based on their density-calibrated weight proportions are shown in Table 6.

Table 6

| | | Stir-fried pepper and meat-1 | Stir-fried pepper and meat-2 | Stir-fried pepper and meat-3 |
|---|---|---|---|---|
| Calibrated weight proportion (%) | Pepper | 59% | 46% | 37% |
| | Pork | 37% | 50% | 59% |
| Calibrated Total Energy (Kcal/100g) | | 97 | 112 | 123 |
| Actual Total Energy (Kcal/100g) | | 88 | 108 | 127 |
| Relative error Calibrated Total Energy | | 10% | 4% | -3% |

The calculation results for the other nutritional values showed a substantially similar accuracy. Hence, this demonstrates that after calibration of the actual weight fractions of the ingredients using the density information, the relative error of the calibrated total energy of the three tested stir-fried pepper and meat dishes A-C were all within the range of ±10%.

In a second experiment, two dishes A and B as shown in FIG. 6 of stir-fried carrot and meat with different proportions of carrot and meat were prepared. The experimental details are in Table 7.

Table 7

|  | Carrot (g) | Pork (g) | Cooking oil (g) | Salt (g) | Actual Total Energy (Kcal/100g) |
|---|---|---|---|---|---|
| Dish A | 100 | 50 | 5 | 1 | 95 |
| Dish B | 75 | 75 | 5 | 1 | 113 |

The recipe information of a default stir-fried carrot and meat dish was retrieved from the recipe database 20 and nutritional information of the listed ingredients was retrieved from the ingredients database 30, with the retrieved information summarized in Table 8. Based on the default weight proportion, the default total energy is 95 Kcal/100g of the dish prepared in accordance with the recipe.

Table 8

|  | Name | Default weight (g) | Default weight proportion (%) | Nutrients | | Density (g/mL) |
|---|---|---|---|---|---|---|
|  |  |  |  | Energy (Kcal/100g) | ... |  |
| Ingredients | Carrot | 200 | 64% | 32 | ... | 0.54 |
|  | Pork | 100 | 32% | 143 | ... | 0.7 |
| Seasonings (e.g., cooking oil, sugar, salt) | Cooking oil | 10 | 3% | 899 | ... |  |
|  | Salt | 2 | 1% | 0 | ... |  |
| Total |  | 312 | 100% | 95 | ... |  |

In this experiment, digital images of the dishes A-B were obtained with a smart phone and the area ratio of different ingredients in these images was analyzed using the similarity algorithm by determining the number of pixels occupied by each ingredient in the dish area of these images. The results are listed in Table 9.

Table 9

|  | Dish A | Dish B |
|---|---|---|
| Area ratio of carrot (%) | 75% | 55% |
| Area ratio of meat (%) | 25% | 45% |
| Total | 100% | 100% |

As previously explained, by assuming that the area ratio of different ingredients is equal to the volume ratio, the weight proportion of different ingredients by weight can be estimated using the density information for the corresponding ingredients as obtained from the ingredient database 30. The calculation results for the energy content of these dishes based on their density-calibrated weight proportions are shown in Table 10.

Table 10

|  |  | Dish A | Dish B |
|---|---|---|---|
| Calibrated weight proportion (%) | Carrot | 67% | 47% |
|  | Pork | 29% | 49% |
| Calibrated Total Energy (Kcal/100g) |  | 88 | 111 |
| Actual Total Energy (Kcal/100g) |  | 95 | 113 |
| Relative error Calibrated Total Energy |  | 7% | 2% |

This again shows that after calibration of the weight fractions based on the density information, the relative error of the calibrated total energy of two tested stir-fried carrot and meat dishes were both within the range of $\pm 10\%$. It is noted that when using the uncalibrated weight proportions of the main ingredients for the calculation of the nutritional value of the dish, a larger relative error resulted.

According to an aspect of the present invention, a computer program product may be provided comprising a computer readable storage medium having computer readable program instructions (code) embodied therewith for, when executed on the processor arrangement 15 of the electronic device 10, cause the processor arrangement 15 to implement any embodiment of the method 100.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. Such a system, apparatus or device may be accessible over any suitable network connection; for instance, the system, apparatus or device may be accessible over a network for retrieval of the computer readable program code over the network. Such a network may for instance be the Internet, a mobile communications network or the like. More specific examples (a non-exhaustive list) of the computer readable storage medium may include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of the present application, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out the methods of the present invention by execution on the processor arrangement may be written in any combination of one or more programming languages, including an object oriented programming language such as Java, Smalltalk, C++ or the like and conventional procedural programming languages, such as the "C" programming language or similar programming languages. The program code may execute entirely on the processor arrangement as a stand-alone software package, e.g. an app, or may be executed partly on the processor arrangement and partly on a remote server. In the latter scenario, the remote server may be connected to the remote device 50 through any type of network, including a local area network (LAN) or a wide area network (WAN), or the connection may be made to an external computer, e.g. through the Internet using an Internet Service Provider.

Aspects of the present invention are described above with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions to be executed in whole or in part on the processor arrangement 15 of the electronic device 10, such that the instructions create means for implementing the functions/acts specified in the flowchart and/or block diagram block or blocks. These computer program instructions may also be stored in a computer-readable medium that can direct the electronic device 10 to function in a particular manner.

The computer program instructions may be loaded onto the processor arrangement 15 to cause a series of operational steps to be performed on the processor arrangement 15, to produce a computer-implemented process such that the instructions which execute on the processor arrangement 15 provide processes for implementing the functions/acts specified in the flowchart and/or block diagram block or blocks. The computer program product may form part of the electronic device 50, e.g. may be installed on the electronic device 10.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

**Claims**

1. A method (100) of estimating a nutritional content of a dish comprising a plurality of ingredients, the method comprising:

   retrieving (107, 109) a list of ingredients of the dish;
   retrieving nutritional information for each of said listed ingredients from an ingredient database;
   receiving (111) a digital image of said dish;
   evaluating (113) the digital image based on the retrieved list of ingredients using ingredient identifiers for each of the ingredients in said list to identify regions within the digital image occupied by said ingredients;
   estimating the relative amounts of the ingredients in said dish recognized in said digital image from said identified regions;
   estimating (115) the nutritional content of said dish using said estimated relative amounts and the retrieved nutritional information; and
   generating (117) an output comprising the estimated nutritional content of said dish.

2. The method of claim 1, wherein identifying regions within the digital image occupied by said ingredients and estimating the relative amounts of the ingredients in said dish recognized in said digital image from said identified regions comprises:

   segmenting the digital image into segments occupied by a single ingredient;
   grouping the segments corresponding to the same ingredient into an ingredient segment group; and
   determining the total area of the dish covered by each ingredient segment group; and wherein:
   determining the relative amounts of said individual ingredients is based on the determined total areas of the ingredient segment groups.

3. The method (100) of claim 2, further comprising:

   obtaining density information for each ingredient;
   converting the determined total areas of each of the ingredient segment groups into total volumes; and wherein:
   determining the relative amounts of each individual ingredient is based on the product of the total volume of the its ingredient segment group and its density.

4. The method (100) of claim 3, wherein the density information corresponds to a particular cooking method of the dish.

5. The method (100) of any of claims 1-4, wherein retrieving said list of ingredients comprises:

   receiving (103) a dish identifier for said dish; and
   retrieving (107) a recipe of said dish from a recipe database upon matching said recipe to said dish identifier, said recipe comprising said list of ingredients and default amounts of said ingredients within the dish, and updating said default amounts based on the estimated relative amounts for said ingredients.

6. The method of claim 5, wherein the dish identifier is a dish name or a dish image.

7. The method (100) of claim 5 or 6, wherein said recipe comprises default relative amounts of one or more seasonings for said dish, the method further comprising estimating the nutritional content of said dish based on the estimated relative amounts of the ingredients and the default relative amounts of one or more seasonings for said dish.

8. The method (100) of claim 7, further comprising:

   receiving a user input updating the relative amount of at least one of said one or more seasonings for said dish; and
   estimating the nutritional content of said dish based on the updated relative amounts of the ingredients, the user-updated amounts of seasonings for said dish and the default relative amounts of the remaining seasonings for said dish.

9. The method (100) of any of claims 2-8, further comprising estimating the relative amount of a single unidentified ingredient in the evaluation of said digital image from a difference between the total area occupied by the dish in said digital image and the total area covered by the identified ingredient segment groups within said dish.

10. The method (100) of any of claims 2-9, further comprising estimating the relative amount of multiple unidentified ingredients in the evaluation of said digital image from:

> a difference between the total area occupied by the dish in said digital image and the total area covered by the identified ingredient segment groups within said dish; and
> a ratio between the amounts of the multiple unidentified ingredients in the recipe.

11. The method (100) of any of claims 1-4, wherein retrieving said list of ingredients comprises parsing (109) a received dish name for individual ingredients in said dish name and generate the list of ingredients from said parsing.

12. The method (100) of any of claims 1-11, wherein the ingredient identifiers include at least one of colour, texture and shape of said ingredient and/or wherein the nutritional content is at least one of total energy content, fat content, carbohydrate content and protein content.

13. A computer program product comprising a computer readable storage medium having computer readable program instructions embodied therewith for, when executed on a processor arrangement (15) of an electronic device (10), causing the processor arrangement to implement the method (100) of any of claims 1-12.

14. An electronic device (10) comprising a processor arrangement (15) and the computer program product of claim 13, wherein the processor arrangement is adapted to execute said computer readable program instructions.

15. The electronic device (10) of claim 14, further comprising at least one of:

> a camera (14) communicatively coupled to the processor arrangement (15) and arranged to capture said digital image; and
> a display device (12) communicatively coupled to the processor arrangement and arranged to display said output.

(A)                                        (B)

**FIG. 1**

14

12

10

**FIG. 2**

20

14

12

10

15

30

**FIG. 3**

100

**FIG. 4**

(A)                    (B)                    (C)

**FIG. 5**

(A)                    (B)

**FIG. 6**

Europäisches
Patentamt

European
Patent Office

Office européen
des brevets

## EUROPEAN SEARCH REPORT

Application Number

EP 20 18 2440

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 9 349 297 B1 (ORTIZ CAONABO ANTHONY [US] ET AL) 24 May 2016 (2016-05-24) * column 11, line 5 - column 20, line 35 * * figure 2 * ----- | 1-15 | INV. G16H20/60 |
| A | US 2016/063692 A1 (DIVAKARAN AJAY [US] ET AL) 3 March 2016 (2016-03-03) * paragraph [0018] - paragraph [0093] * ----- | 1-15 | |

TECHNICAL FIELDS
SEARCHED      (IPC)

G16H

The present search report has been drawn up for all claims

| Place of search | Date of completion of the search | Examiner |
|---|---|---|
| Munich | 26 November 2020 | Rinelli, Pietro |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...............................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 20 18 2440

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2020

| Patent document cited in search report | | Publication date | Patent family member(s) | | Publication date |
|---|---|---|---|---|---|
| US 9349297 | B1 | 24-05-2016 | AU | 2016320586 A1 | 26-04-2018 |
| | | | CN | 108140328 A | 08-06-2018 |
| | | | EP | 3347889 A1 | 18-07-2018 |
| | | | HK | 1257551 A1 | 25-10-2019 |
| | | | JP | 2018528545 A | 27-09-2018 |
| | | | US | 9349297 B1 | 24-05-2016 |
| | | | US | 2017069225 A1 | 09-03-2017 |
| | | | WO | 2017044161 A1 | 16-03-2017 |
| US 2016063692 | A1 | 03-03-2016 | US | 2016063692 A1 | 03-03-2016 |
| | | | US | 2016063734 A1 | 03-03-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016110423 A1